# EUROPEAN PATENT SPECIFICATION

(11) **EP 3 700 188 B1**
(45) Date of publication and mention of the grant of the patent: **19.02.2025**
(21) Application number: 19158220.4
(22) Date of filing: 20.02.2019
(51) Int. Cl.: H04N 23/61, H04N 23/66, H04N 23/67, H04N 23/69, H04N 23/695, A61B 5/00, G08B 13/196, H04N 7/18, H04N 21/422

(54) **SENSOR CONFIGURATION BASED ON OTHER SENSOR CONTEXT DETERMINATION**
SENSORKONFIGURATION AUF BASIS DER KONTEXTBESTIMMUNG ANDERER SENSOREN
CONFIGURATION DE CAPTEUR BASÉ SUR LA DÉTERMINATION DE CONTEXTE D'AUTRES CAPTEURS

(43) Date of publication of application: 26.08.2020
(73) Proprietor: Nokia Technologies Oy, 02610 Espoo (FI)
(72) Inventor: MATHUR, Akhil, Cambridge, CB5 8AG (GB); MONTANARI, Alessandro, Cambridge (GB); LEE, Seungchul, Cambridge (GB); MIN, Chulhong, Cambridge (GB)
(74) Representative: Nokia EPO representatives

(56) References cited:
- WO-A1-2016/081778
- WO-A2-2016/012865
- US-A1- 2016 150 196
- US-A1- 2016 241 768
- US-A1- 2016 306 420
- US-A1- 2017 061 213
- US-A1- 2018 235 538

## Description

### Field

The present application relates to apparatus and methods for sensor configuration based on other sensor context determination, for example, but not exclusively for camera sensor configuration based on wearable sensor context determination.

### Background

Image and video processing technology is a key driver for a number of high-value applications in areas such as industrial automation, self-driving automobiles and public safety systems. To enable these applications, it is common to deploy one or multiple 'high-cost' sensors. By 'high-cost' the disclosure means sensors which require significant power and/or communication bandwidth and/or processing cost to operate. An example of such 'high-cost' sensors is an image camera which may be configured to continuously record and analyse video streams of a scene to extract meaningful information. For instance, a camera mounted inside a self-driving car can be used to analyse the facial expressions of its passengers determine whether the passengers are experiencing anxiety or stress. This information can then be used to control the car's self-driving (AI) system in order to attempt to reduce the stress or anxiety of the passenger, for example to control the car speed to slow the car. In a smart factory, image cameras can monitor the factory floor to look for anomalous events and provide feedback to a human operator or safety system. 'High-cost' sensors may furthermore include sensors which have a limited designed usage lifespan or consume resources which may or may not be able to be replaced when in use. For example a 'high-cost' sensor may be a chemical sensor which uses a chemical reagent to enable detection.

US 2016/0241768 A1 describes controlling the operations of a camera based on one or more sensors attached to one or more actors. Sensor data collected from the sensors is analysed to identify a state of an actor. The state of the actor is used to determine an operation parameter of the camera, such as the zoom level of the camera and/or the direction of the camera, and control the operation of the camera. For example, an actor who is in a state about to perform an interesting action can be selected from a plurality of actors; and the direction and the zoom level of the camera can be adjusted to focus on the selected actor in capture one or more subsequent images.

WO 2016/012865 A2 describes a wearable apparatus and method for processing images including product descriptors. In one implementation, a wearable apparatus for processing images including a product descriptor is provided. The wearable apparatus includes a wearable image sensor configured to capture a plurality of images from an environment of a user of the wearable apparatus. The wearable apparatus also includes at least one processing device programmed to analyse the plurality of images to identify one or more of the plurality of images that include an occurrence of the product descriptor. Based on analysis of the one or more identified images, the at least one processing device is also programmed to determine information related to the occurrence of the product descriptor. The at least one processing device is further configured to cause the information and an identifier of the product descriptor to be stored in a memory. US2017061213 A1 is a similar document which further discloses to determine the emotional environment from facial expressions of a user.

US 2018/0235538 A1 describes a wearable system for monitoring a person's food consumption comprising a motion sensor worn on a person's wrist, a camera worn on the person's ear or housed in the person's eyewear, and a data processor which analyses data from the motion sensor. The camera is automatically triggered to take pictures of food when analysis of data from the motion sensor indicates that the person is eating.

WO 2016/081778 A1 describes the intelligent synchronization and transfer of generally concise event videos synchronized with motion data from motion capture sensor(s) coupled with a user or piece of equipment. It aims to save storage and increases upload speed by uploading event videos and avoiding upload of non-pertinent portions of large videos, to provide intelligent selection of multiple videos from multiple cameras covering an event at a given time (for example selecting one with least shake) and to enables near real-time alteration of camera parameters during an event determined by the motion capture sensor and alteration of playback parameters and special effects for synchronized event videos. It creates highlight reels filtered by metrics and can sort by metric, and integrates with multiple sensors to save event data even if other sensors do not detect the event. It also enables analysis or comparison of movement associated with the same user, other user, historical user or group of users.

US 2016/0150196 A1 describes: providing a threshold; receiving a signal with a camera module, the signal from a tag; determining a location of the tag relative to the camera module based on receiving the signal from the tag, and determining the location includes determining a distance between the tag and the camera module; capturing an image based on the threshold being crossed and the tag being within a frame of the camera module; and recording metadata for the image.

### Summary

There is provided according to a first aspect an apparatus according to claim 1.

There is provided according to a second aspect a method according to claim 6.

There is provided according to a third aspect a computer program according to claim 10.

### Summary of the Figures

For a better understanding of the present application, reference will now be made by way of example to the accompanying drawings in which:
Figure 1 shows schematically a system of apparatus suitable for implementing some embodiments;
Figure 2 shows a flow diagram of the operation of the system as shown in Figure 1 in some embodiments;
Figure 3 shows schematically a further system of apparatus suitable for implementing some embodiments;
Figure 4 shows schematically a first example system suitable for implementing some embodiments;
Figure 5 shows schematically a second example system of apparatus suitable for implementing some embodiments;
Figure 6 shows schematically a third example system of apparatus suitable for implementing some embodiments; and
Figure 7 shows an example device suitable for implementing the apparatus shown in the figures above.

### Embodiments of the Application

The following describes in further detail a suitable system of apparatus and possible mechanisms for the provision of efficient sensor configuration.

The concept may be exemplified by the use of a set (or type) of sensors. This set of sensors may be configurable sensors and may be 'high-cost' sensors. The system may furthermore feature a further set (or type) of sensors. This further set of sensors may be sensors which provide data which is used to control the 'high-cost' set of sensors. The further set (or type) of sensors may be 'low-cost' sensors. A 'low-cost' sensor may be one which has a lower resource consumption or requirement than the 'high-cost' sensor in terms of at least one resource. As such the terms 'low-cost' and 'high-cost' are relative terms. For example a 'low-cost' sensor may still have an enormous cost so long as it is still lower than the 'high-cost' sensor such that its use will still result in an overall lower use of whatever resource we are assessing for a 'cost' parametric. Thus in the example of a limited lifespan sensor a 'low-cost' sensor may be one which has a fixed and non-replaceable power source but has a designed lifespan which is longer than a 'high-cost' sensor.

In some embodiments there can be no difference in price/processing/power consumption between the sets of sensors.

In some embodiments data from the sensor or sensors may be employed to reconfigure the reconfigurable sensor(s). This may be even where the (wearable) sensors from the further set have a greater associated cost factor or where there is no cost factor involved.

In some embodiments the examples allow the system to get the best data out of the reconfigurable sensor.

Examples of 'low-cost' sensors or wearable sensors may be ear-worn devices such as earbuds. The sensors may be employed to dynamically 'sense' an environment and in some embodiments the users within the environment and their operating conditions, and use this information to adapt the operating parameters of the configurable or 'high-cost' (for example camera) sensor system..

In the following examples the configurable or 'high-cost' sensor is a camera system, for example a camera mounted on at least one adjustable gimbal.

Furthermore in the following examples the 'low-cost' sensor is a wearable or personal sensor. The personal sensor may be an example of a sensor configured to monitor a user within their environment. The sensor may be located on or about a user.

For example the sensor may be in direct contact with the user, for example a smart earbud located in the ear of the user. Furthermore in some embodiments the sensor may be affixed to clothing of the user, for example a sensor button worn on a shirt or jumper, or may be attached to a lanyard and worn. In some embodiments the sensor is one which is associated with the user and monitoring the user in their environment without necessarily being located on or worn by the user. For example a non-worn personal sensor may be a mini-drone comprising camera and microphones monitoring the user. The sensor may be configured to act as an external sensor which can anticipate the dynamic context of the environment (e.g., number of people, their current activities) and provide this feedback to the camera system which leads to a real-time reconfiguration of the camera parameters (e.g., field of view, zoom).

Furthermore in some embodiments the further set of sensors may be an assorted or diverse range of sensors. For example the further set of sensors can comprise a number of wearable (smart earbud) sensors which are augmented with other sensors including inertial measurement unit (IMU) sensors, audio sensors, and RF sensors.

In such a manner the example wearable sensors may be able to capture an individual's context of interest and furthermore enable a more energy-efficient system to be implemented when compared to continuous camera recording and processing.

Additionally the embodiments as described hereafter can be employed to provide a more effective detection environment and thus not limited to camera-visible content. In other words users may be monitored out of the camera's view but in locations which the camera may be configured to view, for example by repositioning the camera of changing a level of zoom.

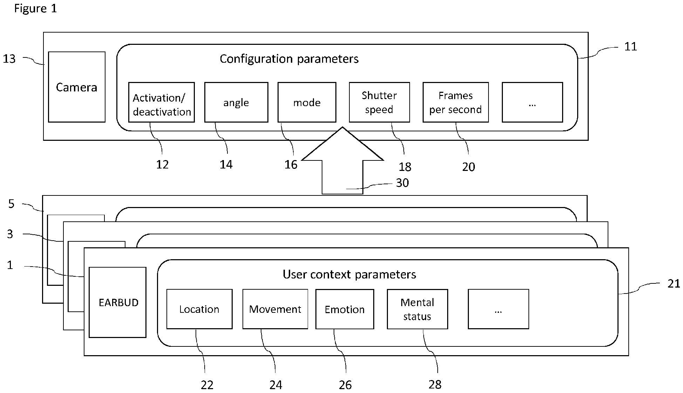

With respect to Figure 1 is shown a schematic view of example apparatus implementing some embodiments. Figure 1 specifically shows a 'high-cost' sensor in the form of a camera system 13. The camera system 13 has a series of configuration parameters 11 which control the operation of the camera system 13. Example configuration parameters 11 can be an activation/deactivation parameter 12 which causes the camera to be active or inactive, an angle (or direction) parameter 14 which controls the camera system to be orientated with a specific field of view, a mode of operation parameter 16 which controls the mode of operation of the camera (for example to control the output resolution of the images), a shutter speed parameter 18 configured to control the shutter speed of the camera system, a frames per second parameter 20 configured to control the number of image frames to be captured per second, and so on. Each of these configuration parameters can be dynamically configured or controlled based on receipt of trigger control messages 30 or other received trigger control signals.

In the example shown in Figure 1 the trigger control message or signals are generated directly by a suitable 'low-cost' wearable sensor. In the example shown in Figure 1 there are shown three earbud ('low-cost') sensors 1, 3, 5 configured to monitor a user context or user context parameter 21 and generate suitable trigger control messages based on the user context parameter 21. However any suitable number of wearable sensors may be employed in a system.

The user context parameters can be determined by the sensor based on one or more sensed parameters, such as pulse, blood pressure, eye-movement or other body related metrics, audio signals, displacement and/or velocity and/or acceleration sensed from the user by the wearable sensor. Examples of user context parameters 21 can be location 22, which determines the location or position of the user within the environment, and movement 24 which characterizes the movement (or activity) of the user. The location 22 and movement user context parameters can be determined based on the sensed displacement and/or velocity and/or acceleration. Other example context parameters 21 may be emotion 26, and mental status 28 which could for example be obtained based on the body related metrics.

These user context parameters 21 can then be used to generate the trigger control messages or signals 30.

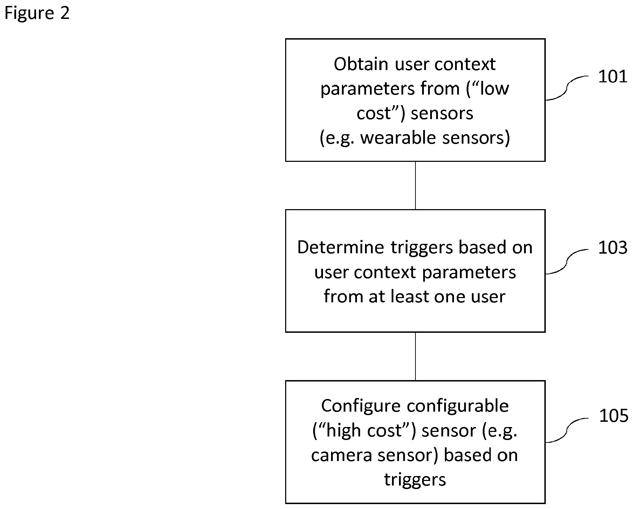

This is shown for example with respect to Figure 2 where a flow diagram shows the operations of the system shown in Figure 1.

Thus for example the earbud (or suitable wearable/low-cost) sensor is configured to obtain at least one user context parameter as shown in Figure 2 by step 101.

Having determined at least one user context parameter then the system can be configured to obtain or determine any triggers based on the obtained at least one user contact parameter from at least one user as shown in Figure 2 by step 103.

Then the camera sensor system, having received the trigger message or signal is configured to dynamically configure the camera sensor based on the trigger message as shown in Figure 2 by step 105.

In this example the obtaining (or generation) of the trigger message occurs within the wearable or personal sensor. However, in some embodiments, the obtaining of the trigger message can be performed elsewhere. For example in some embodiments the wearable sensors ('low-cost' sensors) communicate to the camera ('high-cost') sensor and the trigger messages are obtained internally within the camera ('high-cost') sensor and used to configure the camera ('high-cost') sensor.

Furthermore in some embodiments there may be management or control system apparatus which is configured to receive the sensor information from the (wearable) sensors and from this sensor information (or at least a filtered or processed form suitable for communication over a wireless communication link) the user context parameters and the trigger messages generated and passed to the (camera) configurable sensor.

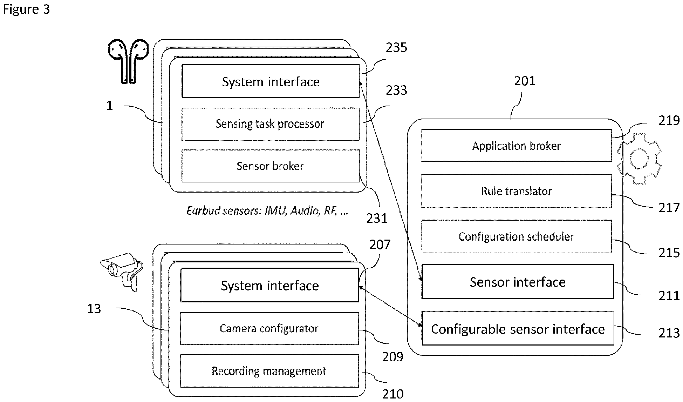

For example Figure 3 shows schematically an example system of wearable sensors, camera sensors and a management or control system apparatus (which is physically separate from camera or earbud sensors) and suitable for implementing the dynamic control of the camera sensor according to some embodiments. It is understood that in some embodiments the management or control system apparatus functions can be implemented in either of, or distributed between, the wearable sensors and the camera sensor.

The management system apparatus 201 can in some embodiments comprise an application broker 219. The application broker 219 can be configured to register the rules of the camera operation. The rules in some embodiments can comprise 2 parts.

The first part of the rule is a user context of interest and the second part of the rule is a corresponding camera configuration or parameter configurations. The user context of interest can in some embodiments be considered to be a context parameter. The user context of interest or context parameter in some embodiments can be specific with respect to a user or a defined group of users (or sensors) or may be generally applicable to all users (or sensors). The context parameter may be defined by one of more sensor signals values. Furthermore the camera configuration may affect one or more camera parameters.

For example a rule may be to highlight a user "Tom" when they look tired, which triggers the camera to locate the user "Tom" within a field of view by changing the angle configuration parameter. In this example the context parameter is the determination of "Tom" looking tired which can be defined in terms of sensor data values representing a lack of motion of "Tom" or head motion sensor values indicating tiredness. The parameter configurations furthermore in this example would be changing the angle configuration parameter to bring "Tom" within the field of view. A further example could be to lower the frame rate per second when it is determined that nothing is moving within a defined area.

In some embodiments the management system apparatus 201 can comprise a rule translator 217. The rule translator 217 can be configured to separate the rule into suitable user contexts of interest and camera configurations.

In some embodiments the management system apparatus 201 can comprise a sensor interface 211. The sensor interface 211 can be configured to operate as an interface between the management system apparatus and the wearable sensors. In some examples the sensor interface is known as an earbud broker configured to operate as a broker between the earbud sensor and management system apparatus. For example where the user contexts are obtained within the wearable sensors the sensor interface211 may be configured to receive the user contexts of interest from the rule translator 217 and pass them to the earbud sensors for the earbud sensors to monitor. Furthermore at runtime when the event or user context of interest is detected by the earbud then the sensor interface 211 may be configured to receive an indicator that the context of interest has been sensed.

In some embodiments the management system apparatus 201 can comprise a configuration scheduler 215 configured to receive via the sensor interface 211 any indicators from the earbud sensors that user context of interest have been detected. The indications from multiple and potentially all of the earbud sensors may be aggregated and then using the rule translator 217 and/or application broker 219 generate suitable camera configuration controls (the trigger messages).

The management system apparatus 201 can comprise a configurable sensor interface 213 configured to control the sending of the configuration control results to the camera 13. In some examples the configurable sensor interface 213 is known as a camera broker configured to operate as a broker between the camera and management system apparatus. The configuration control results can then dynamically change the camera configuration.

With respect to the earbud or wearable sensor 1, the earbud can comprise a sensor broker 231. The sensor broker 231 is configured as an interface with the sensor elements and activate suitable sensor modalities, receive the sensor streams and forward the sensed data to a sensing task processor 231.

The earbud can furthermore comprise a system interface 235. The system interface235 (or system broker as it may also be known in some embodiments) can be configured as the interface with the management system apparatus 201 and configured to receive the user contexts of interest to monitor and pass these to the sensing task processor 231.

Additionally the earbud can comprise a sensing task processor 233. The sensing task processor 233 is configured to receive the sensor data and the user contexts of interest and monitor whether the sensor data matches any of the user contexts of interest. The sensing task processor may implement this in any suitable manner and may comprise sensing modalities, feature extraction and a classifier.

Furthermore when the sensing task processor 233 determines a context of interest from the sensor data then this may be indicated to the system interface 235 which communicates this to the management system apparatus. In some embodiments the functionality of the earbud sensors, for example the sensing task processor, for at least some of the sensors may be offloaded to management system apparatus (or the camera where the functionality of the management system apparatus is implemented in the camera).

With respect to the camera 13 the camera can comprise a system interface 207. The system interface 207 (or system broker as it may also be known in some embodiments) is configured as an interface with the management system apparatus and can be configured to receive a list of configurations from the management system apparatus.

The camera 13 can furthermore comprise a camera configurator 209. The camera configurator can be configured to dynamically change the camera configuration based on a received message from the system broker 207.

Additionally the camera 13 can furthermore comprise a recording management 210. The recording management 210 is configured to manage the recording process.

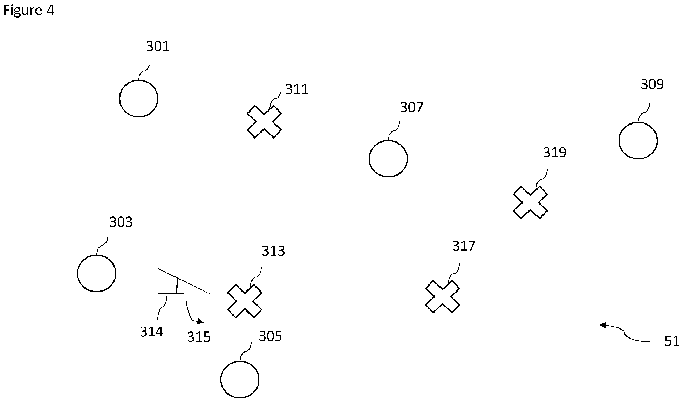

With respect to Figures 4 to 6 is shown a series of practical implementation examples of the apparatus as shown in Figures 1 and 3.

Figure 4 for example shows a system within a factory, industrial or commercial areas 51 configured to monitor the health and safety of the workers or users within the area 51. This for example may be particularly important in areas where employees or users use potentially lethal machines or substances and a prompt response to any accident is crucial.

In this example are shown a series of users for example users 'Annie' 301, 'Bob' 303, 'Carl' 305, 'Doug' 307, and 'Eric' 309 within the area and equipped with a wearable sensor. Additionally within the area 51 there are located a number of cameras 311, 313, 317, 319. These cameras can be configured to dynamically operate based on the trigger messages or signals received from the wearable sensors. Thus for example as shown in Figure 4 the camera 313 has a first field of view 314 and can view user 'Bob' 303 but not 'Carl' 305. However a trigger event generated by the wearable sensor on 'Carl' 305 causes the camera 313 to change its field of view shown by arrow 315 such that the camera 313 can now capture an image in which 'Carl' 305 is visible and can be monitored by the camera.

In such a manner the system shows a way to unobtrusively monitor the workers and be able to trigger a re-configuration of the cameras in the factory to focus on a specific subject or more than one subject simultaneously. In this context, firstly in the application broker 319 the configuration rules can be specified. Example rules may be specific as discussed above for a user. For example 'focus on carl when 'Carl' is working with the power hammer and is showing drowsiness'. Some of the rules could be generic. For example 'zoom-in on anyone who looks dizzy'.

The wearable sensor can in some embodiments be an earbud sensor used to detect the exact user context. For example the earbud can comprise an inertial sensor for motion sensing and microphone for audio sensing. Using this sensor data the earbud can be configured to detect a power hammer by analysing the audio signals (for example through machine learning models). Furthermore the motion sensing can be used to detect drowsiness or dizziness by sensing the motion of a user's head.

The rules can be created with any combination of the contexts detected by the earbuds and the configurations allowed by the cameras.

In some embodiments rules can be chained or combined with one another using suitable logical operators (in other words use of operators AND, OR etc).

Furthermore the location of the user, necessary to move the cameras in the appropriate direction, could be obtained by triangulating the radio beacons emitted by the earbud using static receivers in the area.

In such a manner the embodiments may contribute in that it can be possible to reduce the number of cameras deployed in a factory floor/area given that the available ones can be dynamically re-purposed to cover different areas. Also there may be improvements in that there are potential savings in network bandwidth and power consumption since the cameras can stay in an idle mode, without streaming any data, until an event is detected by the earbuds.

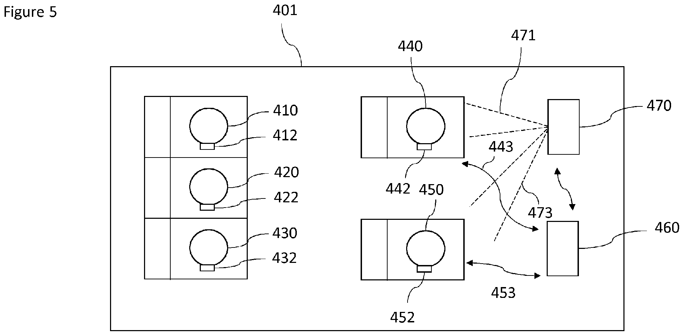

With respect to Figure 5 there is shown in a second example which describes an embodiment of the invention of a practical implementation within which the apparatus shown in Figures 1 and 3 may be implemented. The example shows the interior of an autonomous vehicle 401. The autonomous vehicle 401 comprises a camera 470 configured to monitor the interior of the vehicle. The camera 470 is wirelessly coupled via a management system 460.

The autonomous vehicle 401 furthermore comprises the management system 460 which is configured to control the camera 470 and may also control the autonomous vehicle 401.

Within the autonomous vehicle 401 can be located passengers or users. In the example shown in Figure 5 there are five users and where each of the users are equipped with a wearable or personal sensors (for example a earbud) which is wirelessly coupled to the management system 460 (for example links 443 and 453). Thus for example there may be a first user 410 with a first sensor 412, a second user 420 with a second sensor 422, a third user 430 with a third sensor 432, a fourth user 440 with a fourth sensor 442 and a fifth user 450 with a fifth sensor 452.

The camera 470 can be dynamically configured with a field of view to monitor the users. These images may then be used by the management system 460 to determine whether or not any of the users are experiencing stress and control the vehicle accordingly.

Thus for example as shown in Figure 5 the camera 470 may initially be focusing with a first field of view 471 to view the fourth user 440. However the fifth user 450 with the fifth sensor 452 could be experiencing signs of anxiety which are detected by the sensor 452 (for example, but not claimed, an elevated heart rate causing a user context parameter of interest to be detected), which is communicated via link 453 to the management system 460 which identifies the camera configuration required based on the stored rules to enable a reconfiguration of the camera to have a field of view 471 to view the fifth user as shown by the field of view 473. The camera can then be used to analyse the image containing the fifth user and determine whether or not the image contains any signs of anxiety of the user and change the driving profile of the autonomous vehicle accordingly. In such a manner the facial expressions of the users within the vehicle can provide feedback to the vehicle AI system for self-adaptation., for e.g., if a passenger is looking anxious about the vehicle's AI-assisted driving, the vehicle can give explanations about its decision-making process. Alternately, it can play soothing music to calm the user or perform another user-calming function.

In addition to the above examples of providing critical real-time feedback, facial expression detection can also be used to personalize the in-vehicle user experience (e.g., to select a music playlist based on a user's current emotion).

In some embodiments the earbud or wearable sensor worn by the users can be configured to detect coarse-level facial expressions, using inertial sensors based on the movement of the jaw.

Once a coarse-grained expression is detected by the earbud of a specific user, this trigger triggers a reconfiguration of the vehicle camera, in other words the camera can be rotated, zoomed, and or focussed to place an emphasis or concentrate on that specific user so that a fine-grained facial expression detection operation can be performed.

A benefit of this approach is that as inertial sensors are much cheaper than a camera from an energy-perspective, they are able to continuously analyse and detect 'coarse-grained' facial expressions or anxiety levels of all the passengers in the vehicle. As a result, the energy and bandwidth hungry camera is used sparingly.

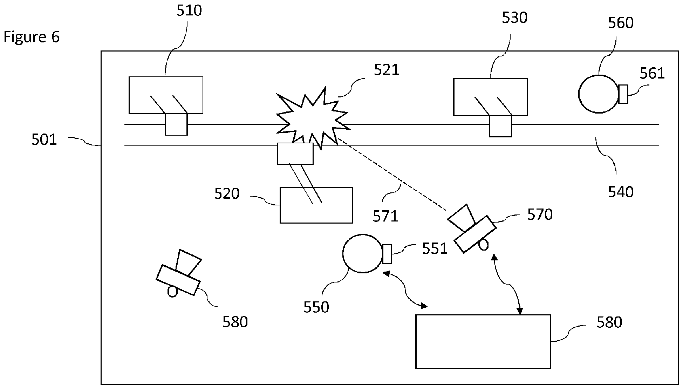

With respect to Figure 6 a third non claimed example is shown whereby the working environment 501 comprises a smart factory production line 540 with a series of machines 510, 520, 530 and their operators 'Fred' 550, and 'Greg' 560. The operators can equipped with suitable wearable or personal sensors 551 (associated with 'Fred' 550) and sensor 561 (associated with 'Greg' 560).

Within the environment there furthermore may be located one or more camera sensors such as shown by the camera sensors 570 and 580. The camera sensors may be dynamically configurable.

Furthermore there may be a management system apparatus 580 in communication with the wearable sensors and the camera.

The example shown in Figure 6 shows a machine 520 about to malfunction (for example generating an abnormal noise 521). The noise as a user context of interest can be detected by one or more of the wearable sensors 551, 561. When an industrial machine is operating it emits specific sounds which are different from the sounds emitted when the operation is not nominal or is about to fail. Using audio-based machine learning models running on the earbuds, an event could be determined when the machine is not producing the nominal sounds. This event can be indicated to the management system 580 and cause a reconfiguration of the camera 570 to view the machine 520 as shown by the dashed line 571 when needed. The images captured by the camera 570 can then be used to determine the potential fault with the machine and therefore may additionally cause a suitable industrial control message to be generated stopping the process, diverting the process to a working machine or alerting users to the potential fault.

In such a manner the microphone on the earbud sensor could be used to infer the status of machines around a user, and cause the cameras to aim in that direction to have a better understanding of what is happening. For example, if a machine malfunction is detected through its audio signature, the system can be configured to focus multiple cameras on this machine, so that a human observer can have a detailed look to determine the potential or actual problem.

In some embodiments the contexts detectable by the earbuds can include also circumstances related to processes happening around the user wearing the earbuds even if where the user is not directly involved (in other words being able to detect faults or potential faults in machines other than the one the user is operating or overseeing).

As such these embodiments allow the monitoring of subtle machine operations which could be very difficult to be monitored by humans, in a non-invasive way in simply leveraging the sound emitted from the machine.

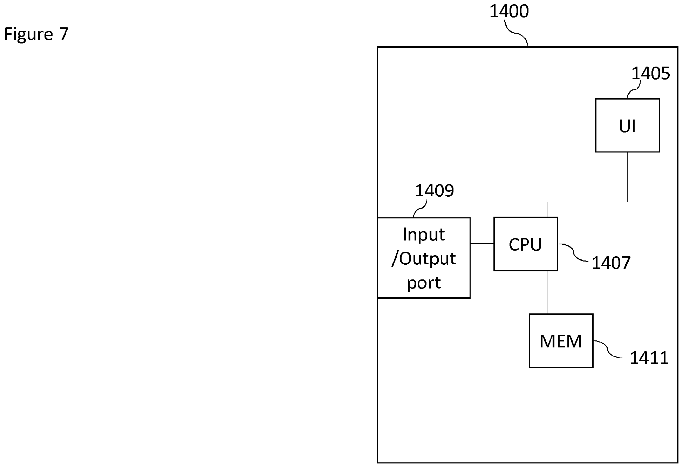

With respect to Figure 7 an example electronic device which may be used as one of the sensors or management system apparatus is shown. The device may be any suitable electronics device or apparatus.

In some embodiments the device 1400 comprises at least one processor or central processing unit 1407. The processor 1407 can be configured to execute various program codes such as the methods such as described herein.

In some embodiments the device 1400 comprises a memory 1411. In some embodiments the at least one processor 1407 is coupled to the memory 1411. The memory 1411 can be any suitable storage means. In some embodiments the memory 1411 comprises a program code section for storing program codes implementable upon the processor 1407. Furthermore in some embodiments the memory 1411 can further comprise a stored data section for storing data, for example data that has been processed or to be processed in accordance with the embodiments as described herein. The implemented program code stored within the program code section and the data stored within the stored data section can be retrieved by the processor 1407 whenever needed via the memory-processor coupling.

In some embodiments the device 1400 comprises a user interface 1405. The user interface 1405 can be coupled in some embodiments to the processor 1407. In some embodiments the processor 1407 can control the operation of the user interface 1405 and receive inputs from the user interface 1405. In some embodiments the user interface 1405 can enable a user to input commands to the device 1400, for example via a keypad/touch interface. In some embodiments the user interface 1405 can enable the user to obtain information from the device 1400. For example the user interface 1405 may comprise a display configured to display information from the device 1400 to the user. The user interface 1405 can in some embodiments comprise a touch screen or touch interface capable of both enabling information to be entered to the device 1400 and further displaying information to the user of the device 1400.

In some embodiments the device 1400 comprises an input/output port 1409. The input/output port 1409 in some embodiments comprises a transceiver. The transceiver in such embodiments can be coupled to the processor 1407 and configured to enable a communication with other apparatus or electronic devices, for example via a wireless communications network. The transceiver or any suitable transceiver or transmitter and/or receiver means can in some embodiments be configured to communicate with other electronic devices or apparatus via a wire or wired coupling.

The transceiver can communicate with further apparatus by any suitable known communications protocol. For example in some embodiments the transceiver can use a suitable universal mobile telecommunications system (UMTS) protocol, a wireless local area network (WLAN) protocol such as for example IEEE 802.X, a suitable short-range radio frequency communication protocol such as Bluetooth, or infrared data communication pathway (IRDA).

The transceiver input/output port 1409 may be configured to receive the signals and in some embodiments determine the parameters as described herein by using the processor 1407 executing suitable code.

These apparatus and mechanisms as discussed above may have the advantage in being able to use a first set of sensors (which may be 'low-cost') in dynamically adapting the operating parameters of configurable sensors (which may be 'high-cost') operating within industrial systems. For example causing dynamic adaptation within a camera-based industrial systems in order to save resources such as network bandwidth and energy consumption. Additionally some embodiments can be employed to increase coverage or effectiveness of the system, for example extending scene coverage, and/or improve the data captured by the system.

Some embodiments may have advantages over sensor systems in that they do not require the deployment of large numbers of configurable sensors, such as image camera-based systems, and are therefore cheaper and more efficient to implement and reduce the amount of resources required to operate the system.

Additionally some embodiments are able to flexibly and dynamically configure the system (such as for a camera: field of view, focus, zoom, resolution). This has a direct improvement on the accuracy and range of data provided by the configurable sensor and therefore may improve the effectiveness of any control system based on the output of the configurable sensor. For example in video processing applications where the camera is within a self-driving car with multiple passengers, the camera can be configured to decide on which passenger to focus on as it may not be possible to focus on all of the passengers at the same time.

In general, the various embodiments of the invention may be implemented in hardware or special purpose circuits, software, logic or any combination thereof. For example, some aspects may be implemented in hardware, while other aspects may be implemented in firmware or software which may be executed by a controller, microprocessor or other computing device, although the invention is not limited thereto. While various aspects of the invention may be illustrated and described as block diagrams, flow charts, or using some other pictorial representation, it is well understood that these blocks, apparatus, systems, techniques or methods described herein may be implemented in, as non-limiting examples, hardware, software, firmware, special purpose circuits or logic, general purpose hardware or controller or other computing devices, or some combination thereof.

The embodiments of this invention may be implemented by computer software executable by a data processor of the mobile device, such as in the processor entity, or by hardware, or by a combination of software and hardware. Further in this regard it should be noted that any blocks of the logic flow as in the Figures may represent program steps, or interconnected logic circuits, blocks and functions, or a combination of program steps and logic circuits, blocks and functions. The software may be stored on such physical media as memory chips, or memory blocks implemented within the processor, magnetic media such as hard disk or floppy disks, and optical media such as for example DVD and the data variants thereof, CD.

The memory may be of any type suitable to the local technical environment and may be implemented using any suitable data storage technology, such as semiconductor-based memory devices, magnetic memory devices and systems, optical memory devices and systems, fixed memory and removable memory. The data processors may be of any type suitable to the local technical environment, and may include one or more of general purpose computers, special purpose computers, microprocessors, digital signal processors (DSPs), application specific integrated circuits (ASIC), gate level circuits and processors based on multi-core processor architecture, as non-limiting examples.

Embodiments of the inventions may be practiced in various components such as integrated circuit modules. The design of integrated circuits is by and large a highly automated process. Complex and powerful software tools are available for converting a logic level design into a semiconductor circuit design ready to be etched and formed on a semiconductor substrate.

Programs, such as those provided by Synopsys, Inc. of Mountain View, California and Cadence Design, of San Jose, California automatically route conductors and locate components on a semiconductor chip using well established rules of design as well as libraries of pre-stored design modules. Once the design for a semiconductor circuit has been completed, the resultant design, in a standardized electronic format (e.g., Opus, GDSII, or the like) may be transmitted to a semiconductor fabrication facility or "fab" for fabrication.

The foregoing description has provided by way of exemplary and non-limiting examples a full and informative description of the exemplary embodiment of this invention. However, various modifications and adaptations may become apparent to those skilled in the relevant arts in view of the foregoing description, when read in conjunction with the accompanying drawings and the appended claims. However, all such and similar modifications of the teachings of this invention will still fall within the scope of this invention as defined in the appended claims.

## Claims

1. A system comprising:
a first wearable sensor (412, 422, 432, 442, 452), being an inertial sensor;
a second sensor (470), comprising a camera being configured to perform image analysis to determine a user's facial expression and being operable to provide second sensor data;
and means for:
obtaining first sensor data from the first sensor (412, 422, 432, 442, 452);
comparing the first sensor data to a context parameter; and causing the second sensor (470) to be dynamically reconfigured based on the comparison to obtain the second sensor data;
wherein:
the first and second sensor data both indicate a facial expression of the user (410, 420, 430, 440, 450) wearing the first sensor (412, 422 ,432, 442, 452), the first sensor data being based on a detected movement of the user's jaw and being a coarser-grain indication of the facial expression than the second sensor data which is based on image analysis; and
use of the second sensor (470) to provide the second sensor data has a higher resource consumption than use of the first sensor (412, 422, 432, 442, 452) to provide the first sensor data.

2. The system of claim 1 wherein the resource consumption is energy consumption.

3. The system as claimed in claims 1 or claim 2, wherein the means for comparing the first sensor data to a context parameter comprises at least one of:
sensing modalities of the first sensor data;
feature extraction of the first sensor data; and
applying a classifier function to the first sensor data.

4. The system as claimed in any of claims 1 to 3, the means for being further for obtaining the context parameter, the context parameter defined by one or more values of the first sensor data.

5. The system as claimed in any preceding claim, wherein causing the second sensor to be dynamically reconfigured based on the comparison comprises at least one of:
causing an activation/deactivation of the camera;
causing a change in an angle of direction of the camera;
causing a change in a field of view/zoom of the camera;
causing a change in a mode of operation of the camera;
causing a change in a shutter speed of the camera; and
causing a change in a frame rate of the camera.

6. A method comprising:
obtaining first sensor data from a first wearable sensor (412, 422, 432, 442, 452), wherein the first sensor is an inertial sensor;
comparing the first sensor data to a context parameter; and
causing a second sensor (470) to be dynamically recontigured based on the comparison to obtain second sensor data;
wherein:
the dynamically reconfigured second sensor (470) comprises a camera that is configured to perform image analysis to determine a user's facial expression and is operable to provide the second sensor data;
the first and second sensor data both indicate a facial expression of a user (410, 420, 430, 440, 450) wearing the first sensor (412, 422, 432, 442, 452), the first sensor data being based on a detected movement of the user's jaw and being a coarser-grain indication of the expression than the second sensor data which is based on image analysis; and
use of the second sensor (470) to provide the second sensor data has a higher resource consumption than use of the first sensor (412, 422, 432, 442, 452) to provide the first sensor data.

7. The method of claim 6 wherein the resource consumption is energy consumption.

8. The method as claimed in claim 6 or claim 7, wherein comparing the first sensor data to a context parameter comprises at least one of:
sensing modalities of the first sensor data;
feature extraction of the first sensor data; and
applying a classifier function to the first sensor data.

9. The method as claimed in any of claims 6 to 8, further comprising obtaining the context parameter, the context parameter defined by one or more values of the first sensor data.

10. A computer program comprising instructions for causing an apparatus to execute the steps of the method of claim 6.

## Patentansprüche

1. System, das Folgendes umfasst:
einen ersten tragbaren Sensor (412, 422, 432, 442, 452), bei dem es sich um einen Inertialsensor handelt;
einen zweiten Sensor (470), der eine Kamera umfasst, die dazu ausgelegt ist, eine Bildanalyse durchzuführen, um den Gesichtsausdruck eines Benutzers zu bestimmen, und betreibbar ist, zweite Sensordaten bereitzustellen;
und Mittel für Folgendes:
Erhalten von ersten Sensordaten vom ersten Sensor (412, 422, 432, 442, 452);
Vergleichen der ersten Sensordaten mit einem Kontextparameter; und
Veranlassen des zweiten Sensors (470) auf Basis des Vergleichs dynamisch neue ausgelegt zu werden, um die zweiten Sensordaten zu erhalten; wobei:
die ersten und die zweiten Sensordaten beide einen Gesichtsausdruck des Benutzers (410, 420, 430, 440, 450) anzeigen, der den ersten Sensor (412, 422, 432, 442, 452) trägt, wobei die ersten Sensordaten auf einer detektierten Bewegung des Kiefers des Benutzers basieren und eine Anzeige gröberer Körnung des Gesichtsausdrucks sind als die zweiten Sensordaten, die auf einer Bildanalyse basieren; und
das Verwenden der zweiten Sensordaten (470), um die zweiten Sensordaten bereitzustellen, einen höheren Ressourcenverbrauch aufweist als das Verwenden der ersten Sensordaten (412, 422. 432, 442, 452), um die ersten Sensordaten bereitzustellen.

2. System nach Anspruch 1, wobei der Ressourcenverbrauch ein Energieverbrauch ist.

3. System nach Anspruch 1 oder Anspruch 2, wobei die Mittel zum Vergleichen der ersten Sensordaten mit einem Kontextparameter mindestens eines von Folgendem umfassen:
Erfassungsmodalitäten der ersten Sensordaten;
Merkmalsextrahiererung der ersten Sensordaten; und
Anwenden einer Klassifiziererfunktion auf die ersten Sensordaten.

4. System nach einem der Ansprüche 1 bis 3, wobei die Mittel ferner dem Erhalten des Kontextparameters dienen, wobei der Kontextparameter von einem oder mehreren Werten der ersten Sensordaten definiert wird.

5. System nach einem der vorhergehenden Ansprüche, wobei das Veranlassen des zweiten Sensors, auf Basis des Vergleichs dynamisch neu ausgelegt zu werden, mindestens eines von Folgendem umfasst:
Veranlassen einer Aktivierung/Deaktivierung der Kamera;
Veranlassen einer Änderung eines Winkels einer Richtung der Kamera;
Veranlassen einer Änderung eines Sichtfeldes/Zooms der Kamera;
Veranlassen einer Änderung eines Betriebsmodus der Kamera;
Veranlassen einer Änderung einer Verschlussgeschwindigkeit der Kamera; und
Veranlassen einer Änderung einer Bildfrequenz der Kamera.

6. Verfahren, das Folgendes umfasst:
Erhalten von ersten Sensordaten von einem ersten tragbaren Sensor (412, 422, 432, 442, 452), wobei der erste Sensor ein Inertialsensor ist;
Vergleichen der ersten Sensordaten mit einem Kontextparameter; und
Veranlassen eines zweiten Sensors (470), auf Basis des Vergleichs dynamisch neu ausgelegt zu werden, um zweite Sensordaten zu erhalten;
wobei:
der dynamisch neu ausgelegte zweite Sensor (470) eine Kamera umfasst, die dazu ausgelegt ist, eine Bildanalyse durchzuführen, um einen Gesichtsausdruck eines Benutzers zu bestimmen, und betreibbar ist, die zweiten Sensordaten bereitzustellen;
die ersten und die zweiten Sensordaten beide einen Gesichtsausdruck eines Benutzers (410, 420, 430, 440, 450) anzeigen, der den ersten Sensor (412, 422, 432, 442, 452) trägt, wobei die ersten Sensordaten auf einer detektierten Bewegung des Kiefers des Benutzers basieren und eine Anzeige gröberer Körnung des Ausdrucks sind als die zweiten Sensordaten, die auf einer Bildanalyse basieren; und
das Verwenden der zweiten Sensordaten (470), um die zweiten Sensordaten bereitzustellen, einen höheren Ressourcenverbrauch aufweist als das Verwenden der ersten Sensordaten (412, 422. 432, 442, 452), um die ersten Sensordaten bereitzustellen.

7. Verfahren nach Anspruch 6, wobei der Ressourcenverbrauch ein Energieverbrauch ist.

8. Verfahren nach Anspruch 6 oder Anspruch 7, wobei das Vergleichen der ersten Sensordaten mit einem Kontextparameter mindestens eines von Folgendem umfasst:
Erfassungsmodalitäten der ersten Sensordaten;
Merkmalsextrahiererung der ersten Sensordaten; und
Anwenden einer Klassifiziererfunktion auf die ersten Sensordaten.

9. Verfahren nach einem der Ansprüche 6 bis 8, das ferner das Erhalten des Kontextparameters umfasst, wobei der Kontextparameter von einem oder mehreren Werten der ersten Sensordaten definiert wird.

10. Computerprogramm, das Anweisungen zum Veranlassen einer Vorrichtung, die Schritte des Verfahrens von Anspruch 6 durchzuführen, umfasst.

## Revendications

1. Système comprenant :
un premier capteur (412, 422, 432, 442, 452) à porter, qui est un capteur inertiel ;
un deuxième capteur (470) comprenant une caméra configurée pour effectuer une analyse d'image afin de déterminer une expression faciale d'un utilisateur, et pouvant fonctionner pour fournir des données de deuxième capteur ;
et des moyens pour :
obtenir des données de premier capteur du premier capteur (412, 422, 432, 442, 452) ;
comparer les données de premier capteur à un paramètre de contexte ; et
provoquer une reconfiguration dynamique du deuxième capteur (470) sur la base de la comparaison, afin d'obtenir les données de deuxième capteur ;
dans lequel :
les données du premier et du deuxième capteur indiquent une expression faciale de l'utilisateur (410, 420, 430, 440, 450) portant le premier capteur (412, 422, 432, 442, 452), les données de premier capteur étant basées sur un déplacement détecté de la mâchoire de l'utilisateur, et donnant une indication à grain plus grossier de l'expression faciale que les données de deuxième capteur qui sont basées sur une analyse d'image ; et
l'utilisation du deuxième capteur (470) pour fournir les données de deuxième capteur consomme plus de ressources que l'utilisation du premier capteur (412, 422, 432, 442, 452) pour fournir les données de premier capteur.

2. Système de la revendication 1, dans lequel la consommation de ressources est une consommation d'énergie.

3. Système selon la revendication 1 ou la revendication 2, dans lequel les moyens pour comparer les données de premier capteur à un paramètre de contexte comprennent au moins un parmi :
des modalités de détection des données de premier capteur ;
l'extraction de caractéristiques des données de premier capteur ; et
l'application d'une fonction de classification aux données de premier capteur.

4. Système selon l'une des revendications 1 à 3, où les moyens sont en outre destinés à obtenir le paramètre de contexte, le paramètre de contexte étant défini par une ou plusieurs valeurs des données de premier capteur.

5. Système selon l'une des revendications précédentes, dans lequel la provocation de la reconfiguration dynamique du deuxième capteur sur la base de la comparaison comprend au moins l'une parmi :
la provocation d'une activation/désactivation de la caméra ;
la provocation d'une modification d'un angle de direction de la caméra ;
la provocation d'une modification d'un champ de vision/zoom de la caméra ;
la provocation d'une modification d'un mode de fonctionnement de la caméra ;
la provocation d'une modification d'une vitesse d'obturation de la caméra ; et
la provocation d'une modification d'une fréquence de trame de la caméra.

6. Procédé comprenant :
l'obtention de données de premier capteur d'un premier capteur (412, 422, 432, 442, 452) à porter, dans lequel le premier capteur est un capteur inertiel ;
la comparaison des données de premier capteur à un paramètre de contexte ; et
la provocation d'une configuration dynamique d'un deuxième capteur (470) sur la base de la comparaison, afin d'obtenir des données de deuxième capteur ;
dans lequel :
le deuxième capteur (470) reconfiguré dynamiquement comprend une caméra configurée pour effectuer une analyse d'image afin de déterminer une expression faciale d'un utilisateur, et peut fonctionner pour fournir les données de deuxième capteur ;
les données du premier et du deuxième capteur indiquent une expression faciale d'un utilisateur (410, 420, 430, 440, 450) portant le premier capteur (412, 422, 432, 442, 452), les données de premier capteur étant basées sur un déplacement détecté de la mâchoire de l'utilisateur, et donnant une indication à grain plus grossier de l'expression que les données de deuxième capteur qui sont basées sur une analyse d'image ; et
l'utilisation du deuxième capteur (470) pour fournir les données de deuxième capteur consomme plus de ressources que l'utilisation du premier capteur (412, 422, 432, 442, 452) pour fournir les données de premier capteur.

7. Procédé de la revendication 6, dans lequel la consommation de ressources est une consommation d'énergie.

8. Procédé selon la revendication 6 ou la revendication 7, dans lequel la comparaison des données de premier capteur à un paramètre de contexte comprend au moins l'un parmi :
des modalités de détection des données de premier capteur ;
l'extraction de caractéristiques des données de premier capteur ; et
l'application d'une fonction de classification aux données de premier capteur.

9. Procédé selon l'une des revendications 6 à 8, comprenant en outre l'obtention du paramètre de contexte, le paramètre de contexte étant défini par une ou plusieurs valeurs des données de premier capteur.

10. Programme informatique comprenant des instructions pour amener un appareil à exécuter les étapes du procédé de la revendication 6.
